# EUROPEAN PATENT APPLICATION

(11) **EP 0 745 849 A2**
(43) Date of publication of application: **04.12.1996**
(21) Application number: 96103448.5
(22) Date of filing: 06.03.1996
(51) Int. Cl.: G01N 33/50

(54) **Sample processing method for whole blood**

(30) Priority: 10.03.1995 US 402284
(71) Applicant: Becton Dickinson and Company, Franklin Lakes, New Jersey 07417-1880 (US)
(72) Inventor: Sigler Dey, Margaret, Apex, North Carolina 27502 (US); Spears, Patricia Anne, Raleigh, North Carolina 27615 (US); Keating, William Edward, Baltimore, Maryland 21234 (US)
(74) Representative: Rambelli, Paolo

(57) **Abstract**

Methods for processing whole blood for nucleic acid analysis of microorganisms which may be present are provided. The methods eliminate inhibitors which interfere in particular with enzymatic nucleic acid reactions and are also compatible with conventional culturing techniques. It has been found that selective lysis of red blood cells with certain lysis buffers, followed by centrifugation at 5,000-15,000 xg for 5-30 min. and washing with water, saline, physiological buffers, or buffers compatible with nucleic acid analysis removes inhibitors of nucleic acid reactions which are present in whole blood or are introduced by reagents used in the sample processing protocol. The method therefore allows nucleic acid analysis of large samples of whole blood without interference from inhibitors, thereby improving the sensitivity of the nucleic acid analysis.

## Description

### FIELD OF THE INVENTION

The present invention relates to methods for preparing biological samples for analysis. In particular the invention relates to sample processing methods for whole blood which are compatible with subsequent nucleic acid analysis, for example nucleic acid amplification.

### BACKGROUND OF THE INVENTION

Nucleic acid-based genetic methods for identification of microorganisms have greatly reduced the time and labor involved in clinical diagnosis. Such methods include, for example, nucleic acid hybridization (e.g., Southern and slot blots), nucleotide sequencing, nucleic acid cloning techniques, restriction digestion of nucleic acids and nucleic acid amplification. In particular, nucleic acid amplification has provided means for rapid, sensitive and specific identification of microorganisms by amplification and detection of specific genes or gene fragments. For use as diagnostic methods, it is of particular interest to apply these nucleic acid analyses to whole blood samples. Prior to the availability of nucleic acid-based methods for detection and identification of microorganisms, whole blood was analyzed for the presence of microorganisms or yeast by culturing. However, processing of clinical samples for nucleic acid analyses requires different criteria than sample processing for culturing: 1) nucleic acids must be released from the microorganism in a form suitable for the analysis, 2) nucleic acids must be present in a composition with the appropriate components, ionic strength and pH for the biochemical reactions of the analysis, and 3) inhibitors of the reactions, if present in the clinical sample or introduced during sample processing, must be removed or rendered non-inhibitory. For nucleic acid amplification, certain inhibitors are known to be present in biological samples. Such inhibitors are of particular concern when the sample to be analyzed is whole blood, which is known to contain several substances which are highly inhibitory to nucleic acid-based diagnostic methods. e.g., hemoglobin, hemin and polysaccharides.

At the present time nucleic acid-based methods for diagnosis and identification of microorganisms do not generally completely replace conventional culturing, as samples which are positive by molecular diagnostic methods are generally cultured to determine drug sensitivity or to verify the positive result. The need for both conventional culturing and genetic analysis from a single sample has demonstrated that conventional processing of whole blood not only introduces inhibitors of subsequent nucleic acid-based reactions, particularly amplification, but also may cause inhibition of growth of microorganisms in culture. Attempts have been made to remove such inhibitors after processing of the blood sample and prior to nucleic acid analysis or culturing. However, the conventional phenol/chloroform extraction methods for removing inhibitors require extensive time and may leave traces of these reagents, which are themselves inhibitory. In addition, residual chaotropes, alcohol or silica, which are conventionally used for purification of nucleic acids may inhibit nucleic acid amplification reactions. The presence of inhibitors in samples processed by conventional methods has also limited the volume of processed sample which can be amplified. That is, to ensure sufficient dilution of inhibitors, only small aliquots of whole blood (usually no more than about 0.10 ml) could previously be processed for nucleic acid amplification. Larger sample volumes produced erratic results or amplification failures. Even amplification of less than 0.10 ml of conventionally processed whole blood samples have produced erratic results in amplification reactions due to the presence of inhibitors in blood or introduced by sample processing.

Certain prior art whole blood processing methods employ lysis of red blood cells as an initial step. However, these methods are generally directed to isolation of white blood cell DNA or tie DNA of organisms found within white blood cells. Therefore, the lysis conditions are sufficiently harsh to lyse extracellular microorganisms present in the blood. These methods are not suitable for obtaining microorganism nucleic acids for analysis unless the microorganisms are in white blood cells. For example, L. Lin, et al. (*Applications Methods* Chapter 8.1, pages 605-616) describe recovery of nuclei of white blood cells by lysing with high concentrations of TRITON or ZAPOGLOBIN. Not only are these reagents highly inhibitory to nucleic acid amplification, microorganisms in the blood will be lysed along with the red blood cells. Microorganism DNA is then lost with the supernatant or in the washes. Similarly, S. Gustincich, et al. (1991. *BioTechniques* 11:298-301) describe extraction of blood cell DNA by first lysing with 8% DTAB. DTAB is very inhibitory to amplification and also lyses any microorganisms present in the blood sample. In general, attempts to process whole blood for nucleic acid analysis of microorganisms lack the sensitivity necessary for diagnostic tests as a result of small sample volumes or dilution to reduce inhibition (e.g., R. H. Barker, et al. 1992 *AM. J. Trop. Med . Hyg.* 46:416-426). Most employ lysis reagents which are highly inhibitory to amplification (e.g., M. Maass, et al. 1992. *Trop. Med. Parasitol.* 43:191-194).

The ideal sample processing method for nucleic acid amplification of microorganisms present in whole blood therefore has the following features: 1) removal of amplification inhibitors, in particular those introduced by lysis of red blood cells, 2) release of a sufficient amount of DNA from the microorganisms for amplification, and 3) the ability to process large sample volumes to improve detection sensitivity. As described above, others have attempted to remove amplification inhibitors introduced by conventional whole blood processing methods either by isolating the nucleic acids from the sample prior to nucleic acid analysis or diluting the processed sample to reduce the effect of inhibitors. Still other conventional protocols for nucleic acid analysis of whole blood rely on small initial volumes of sample (2-100 µl) to reduce inhibitors to an acceptable level. Isolation of nucleic acids is cumbersome and to be effective requires that a high concentration of nucleic acid be present. Dilution or use of small sample volumes significantly compromises the sensitivity of the nucleic acid analysis.

### SUMMARY OF THE INVENTION

The present invention provides methods for processing whole blood samples which are compatible with both conventional culturing techniques and nucleic acid analysis. The methods are based on the discovery of reagents and procedures which can be used to selectively lyse red blood cells without substantial lysis of the cells of microorganisms in tie blood sample. Microorganism nucleic acid to be subsequently analyzed is therefore protected within the cell and can be separated from the lysed red blood cell supernatant for further sample processing. The microorganisms are maintained substantially intact through washing and other sample processing steps. The nucleic acids are then released for subsequent analysis.

It has also unexpectedly been found that inhibition can be significantly reduced or eliminated by selection of the lysing reagent (e.g., saponin), concentration of cell debris and microorganisms, removal of the supernatant, and washing with saline, water, a physiological buffer (e.g., phosphate buffered saline, "PBS") or a buffer compatible with the nucleic acid analysis (e.g., TRIS, phosphate, borate or acetate buffers). The sample may optionally be treated with enzymes or chelators to further reduce or inactivate inhibitory substances and increase the release of target DNA from microorganisms, if necessary. Heating or mechanical disruption of the cells also assists in releasing microorganism nucleic acids at the desired time.

The inventive methods are compatible with both culturing and nucleic acid analysis, allowing a single whole blood sample to be processed for both uses without the need for separate sample processing protocols. The present methods are simpler, faster or more reproducible than previous methods for processing of whole blood for nucleic acid analysis. Reproducibility was previously only possible using time consuming and labor intensive sample processing methods. In addition, no specialized equipment is required for the practice of the invention. The reagents are inexpensive and readily available, and none require special handling. Unlike previous methods, in which the volume of the blood sample which could be analyzed was often limited by the presence of inhibitors, significantly larger volumes may be processed by the inventive methods and reproducibly amplified. Nucleic acid analysis of microorganisms in whole blood processed by the inventive methods therefore has improved sensitivity as compared to nucleic acid analysis of microorganisms in whole blood processed by prior art methods. The ability to amplify from large sample volumes allows the practitioner to detect rare target sequences which may be missed when a small aliquot of a sample or a diluted sample must be amplified to avoid interference from inhibitors.

### DETAILED DESCRIPTION OF THE INVENTION

The present invention provides methods for processing whole blood samples for detection and identification of microorganisms. The sample processing methods are compatible with both culture and nucleic acid-based analysis, particularly nucleic acid analyses requiring enzymatic processes. These include, for example, restriction digestion, nucleotide sequencing, and nucleic acid amplification. The present methods are compatible with a variety of nucleic acid-based reactions because, in contrast to previously known whole blood processing methods, inhibitory substances present in blood samples or introduced by lysis reagents are efficiently removed or eliminated by the sample processing protocol. The present methods are therefore compatible with a variety of protocols for analysis and detection of nucleic acid which are sensitive to inhibition by components of whole blood.

The sample to be processed for analysis of microorganism nucleic acids is whole blood. Because of the efficiency of the inventive methods in removing associated inhibitors of nucleic acid analysis, the whole blood sample may be as large as about 5 ml. This volume of whole blood represents approximately a 50-fold increase in the volume of whole blood which could be analyzed using sample processing methods of the prior art. Lytic reagents are added to the sample in an amount and for a period of time sufficient to lyse red blood cells contained in the sample. TRITON X-100 is an effective lytic reagent, but may be inhibitory to amplification as well as incompatible with culture. It has been found, however, that TRITON X-100, used at a minimal concentration effective for lysis (generally about 0.2-1.0%) in a solution which does not contain sucrose, does not result in inhibition of amplification reactions. The negative effects of TRITON lytic reagents in conventional sucrose solutions were not previously appreciated, however, the present studies have demonstrated that 1% TRITON X-100 in saline allows a five-fold increase in amplification as compared to 1% TRITON X-100 in 320 mM sucrose. BACTEC Lysis Media was found to be totally inhibitory in subsequent amplification reactions, however, the lytic component of BACTEC Lysis Media (saponin) was highly effective and non-inhibitory when used alone (i.e., in phosphate buffered saline or sucrose, without the additional components of BACTEC Lysis Media). Saponin (0.2-0.5% v/v) is a preferred lytic reagent, as it provides improved retention of microorganisms when culturing or viability studies are also desired. To obtain the desired final concentration, saponin is typically added to the sample as a more concentrated stock solution at a ratio of 1:4-1:10 of the sample (v/v). TRIS-EDTA, 8% CTAB, water and SDS were not sufficiently effective lytic reagents. Without wishing to be limited to any particular mechanism by which the invention works, Applicants believe that lysis of red blood cells assists in removing inhibitory substances present in whole blood by allowing red blood cell components to be poured off at a later step of the protocol.

Selective lysis of red blood cells in the sample, as described above, also provides improved recovery of microorganims and therefore improved sensitivity in the nucleic acid analysis. This is most likely due to the fact that the lysis procedure does not substantially lyse either extracellular microorganisms or macrophages, which may contain intracellular microorganisms. Macrophages are likely provide additional protection to microorganisms, preventing their destruction during subsequent steps of the sample processing procedure and improving organism recovery. This is of particular importance when the nucleic acid of a relatively sensitive microorganism, e.g., *E. coli*, is to be analyzed. Red blood cells in samples containing less sensitive microorganisms, such mycobacteria, may be lysed using harsher chemical procedures, e.g., NaOH. Although NaOH also lyses macrophages and releases intracellular mycobacteria, these microorganisms are highly resistant to the subsequent chemical treatments of the sample processing protocol and there is no significant reduction in recovery. Because the method of the invention can maintain macrophages intact with intracellular microorganisms, it also provides a convenient means for separating extracellular microorgansims and intracellular microorganisms for separate nucleic acid analysis

The cell debris in the lysed blood sample is then concentrated. Cell debris includes particulate blood components which are not lysed, e.g., residual red blood cells, platelets white blood cells and microorganisms. Concentration is generally achieved by high speed centrifugation, but filtration can be suitable for less sensitive microorganisms such as mycobacteria. If centrifugation is used to concentrate the cell debris, the speed of centrifugation is critical to obtain efficient removal of inhibitors. At least 5,000 xg is required for efficient concentration and the sample should be centrifuged for a period of time sufficient to form a tight pellet of cell debris which is resistant to disturbance when the supernatant (containing a large proportion of the inhibitors is removed. The sample is preferably centrifuged at about 8,000-15,000 xg for 5-30 min. at about 4°C to maximize recovery of microorganisms from the sample (about 97% recovery in many cases). A standard clinical centrifuge is generally inadequate for this purpose, and a high-speed refrigerated centrifuge is preferred.

The supernatant, containing most of the released red blood cell components and hemoglobin, is then removed with minimal disturbance of the pellet of cell debris. In this manner, microorganisms present in the blood sample are concentrated into a significantly reduced sample volume in the pellet. It was unexpected that releasing red blood cell components known to inhibit subsequent enzymatic reactions by lysis would give better removal than keeping red blood cells intact and removing them from the sample. The pellet is then resuspended for washing in a small volume of water, saline, a physiological buffer, or a buffer compatible with the selected method of nucleic acid analysis. Washing removes additional inhibitors. Preferably, the wash solution is similar to the buffer used in the subsequent nucleic acid analysis. For example, if the nucleic acid is to be hybridized to a probe or sequenced, the wash solution may be the hybridization or sequencing buffer. If the nucleic acid is to be amplified, the wash solution may be a buffer appropriate for the selected nucleic acid amplification reaction. SDA is preferred for amplification of nucleic acids released according to the invention because of the simplicity of this method, and KPDG buffer (32.48 mM KPO₄ pH 7.6, 7.5% DMSO, 3% glycerol) is therefore a preferred wash solution. This buffer is customarily employed in SDA reactions. The volume of the wash solution is generally kept small (e.g., 0.5-1.5 ml) for ease of sample handling. This has the advantage of reducing the volume of potentially biohazardous material which must be disposed of and allows the procedure to be easily performed in a biological safety cabinet. However, larger volumes of wash solution may remove inhibitors more efficiently.

After mixing the cell debris pellet with the wash solution, the sample is centrifuged at a minimum of about 8,000 xg, preferably 8,000-15,000 xg, to produce a tight pellet and prevent loss of material upon further handling. As the sample volume has been significantly reduced at this point of sample processing, washing centrifugations are most conveniently accomplished in a microcentrifuge, for example at 8,000-15,000 xg at room temperature. Centrifugation will generally be for about 1-10 min., preferably about 2-5 min. when the microcentrifuge is used This process can result in essentially 100% recovery of microorganisms. The microcentrifuge also allows centrifugation to be performed in a biological safety cabinet or hood. The supernatant is discarded and, optionally, wash solution is added a second time and mixed with the pellet. The sample is centrifuged as before to form a tight pellet, and the supernatant is discarded. A third wash, performed in a similar manner, may be desirable in some cases. The final pellet is resuspended in a reduced volume (relative to the original sample volume) of water, saline, a physiological buffer, or a buffer compatible with nucleic acid analysis, further concentrating microorganisms for detection or identification. The final volume of the washed and resuspended pellet is typically about 1/10 to 1/20 of the initial volume of the whole blood sample. Prior art methods to concentrate and reduce sample volume to this extent have been met with a concominant increase in amplification inhibition. In contrast, nearly 100% efficiency of amplification can be achieved by lysing and washing whole blood samples as described above.

Washing reduces background DNA, proteins and other low molecular weight components which are inhibitory to nucleic acid analysis and also exchanges the buffer for compatibility with the nucleic acid analysis. One wash is generally sufficient for removal of inhibitors for more robust amplification methods such as PCR or thermophilic SDA. Two or three washes are preferred when the sample is to be amplified in a reaction such as conventional SDA (i.e., lower temperature SDA), which is relatively more sensitive to inhibitors. Optionally, additional washes may be employed as long as the sample is centrifuged at a high enough speed to form a tight pellet which is resistant to loss of material when the supernatant is removed.

In many cases, the foregoing steps are sufficient to remove inhibitors and nucleic acids may be released from the microorganisms for analysis. However, additional optional sample processing steps may be employed to further reduce the effect of inhibitors, thereby improving the sensitivity of the nucleic acid analysis. These include treatment of the lysed, washed, concentrated sample with protease (e.g., Proteinase K, trypsin, lysozyme), a chelator (e.g., EDTA, CHELEX) or nuclease (e.g., RNase A, DNase I). Procedures for using these reagents to remove or inhibit proteins and nucleic acids in a sample are well known, including the appropriate concentrations and incubation periods to obtain the desired result. Proteinase K (0.2-5 mg/ml) is a preferred optional treatment for removing additional small molecules and inhibitors, and may in some cases actually increase levels of amplification. When Proteinase K treatment is used, a sensitivity of 40 microorganisms in 5 ml of whole blood has been observed in thermophilic Strand Displacement Amplification (SDA), corresponding to two target molecules in the amplification reaction. The sensitivity of conventional (lower temperature) SDA using samples processed according to the invention is about 25 microorganisms in 5 ml of whole blood. If necessary, release of nucleic acids from microorganisms may be enhanced by treatments which disrupt the cells. For example, the release of nucleic acids may be improved by mechanical disruption (e.g., glass beads, sonication) of cells or by heating. These and equivalent procedures for disruption of cells are well known.

Heating the sample after treatment with protease, nuclease, chelators or after cell disruption is also optional, but is particularly advantageous when enzyme treatment is employed and the subsequent nucleic acid analysis begins with denaturation of the nucleic acid (e.g., amplification or hybridization). Heating inactivates the enzyme while simultaneously denaturing double stranded nucleic acids in preparation for the analysis. The sample may be heated to about 90-110°C for about 1-20 min. in a water bath, convection oven or by other suitable means. Heating also has the advantage of rendering microorganisms non-viable and non-infectious, and assists in releasing nucleic acids. As the processed blood sample is already in a buffer or solution compatible with the subsequent nucleic acid analysis, after heating it is only necessary to bring the sample to the appropriate temperature and add any additional reagents for the nucleic acid analysis. In the case of amplification, heating denatures the target nucleic acid as well as inactivating optional sample treatment enzymes, so it is only necessary to reduce the temperature and add heat-sensitive enzymes such as polymerase to begin the amplification reaction. If the nucleic acid analysis is for detection or identification of mycobacteria, the heating step also assists in lysing the mycobacteria and releasing nucleic acids.

DNA purification methods have been used in the prior art in attempts to isolate released DNA from inhibitors of nucleic acid analyses. Purification of the nucleic acids released by the blood processing methods of the invention is not necessary, and should generally be avoided as many of the best-known nucleic acid isolation methods contribute to inhibition of amplification reactions. For example, commercially available nucleic acid isolation kits such as ISOQUICK and GNOME result in isolated nucleic acid preparations which are highly inhibitory in amplification reactions. However, if purification of the released nucleic acids is desired, the inventive methods are compatible with certain conventional DNA purification protocols such as phenol/chloroform extraction. In some cases, phenol/chloroform extraction may result in a limited increase in amplification.

If only nucleic acid analyses are to be performed, the entire concentrated pellet of cell debris obtained after the lysis step may be used in the analysis. Alternatively, a portion of the pellet (removed prior to heating) may be used for culture and a portion for nucleic acid analysis, allowing both procedures to be performed on a single sample. In one embodiment, the final pellet from the washing process may be split, prior to heating, and a portion used for culture and a portion processed for nucleic acid analysis. Preferably the portion of the pellet for culture is removed after the first centrifugation step. Alternatively, the lysed blood sample itself may be split, with one portion being centrifuged for culture and the other portion being centrifuged and washed for nucleic acid analysis.

The released nucleic acid in the processed blood sample may then be used in the selected nucleic acid analysis or detection protocol without further treatment, as inhibitory substances are significantly reduced or eliminated by the sample processing methods of the invention. Nucleic acids prepared according to the invention are compatible with any of the known nucleic acid analysis and detection protocols, but the methods of the invention have particular advantages in preparing nucleic acids for use in enzymatic analyses. These include, but are not limited to, restriction digestion and cloning, nucleotide sequencing and nucleic acid amplification. Such protocols are well known in the art and are reviewed in Molecular Cloning: A Laboratory Manual, Second Edition, by J. Sambrook, E. F. Fritsch and T. Maniatis, Cold Spring Harbor Laboratory Press, 1989. The present sample processing methods are particularly useful for amplification of nucleic acids because elimination of inhibitors enhances sensitivity of diagnostic tests and allows the practitioner to amplify a larger volume of the nucleic acid preparation than was previously possible. A target sequence which is extremely rare is therefore more likely to be represented in the aliquot of sample amplified, improving the accuracy and reliability of the amplification reaction.

Certain steps of the present methods have been found to be particularly important to its success. First, the concentrated cell debris pellet formed after lysis of red blood cells must be washed to remove the inhibitory substances present in whole blood or introduced by reagents used in the lysis procedure. A single washing step may not be sufficient for processing many samples. Larger volumes of wash solution, when feasible, result in even more efficient removal of these reagents but are generally not necessary. Most importantly, the sample must be centrifuged after lysis and washing so as to form a pellet which will remain intact during removal of the supenatant and re-washing, e.g., at about 5,000-15,000 xg for 5-30 min. after lysis and about 8,000-15,000 xg for 1-10 min. after each wash.

The following experimental examples are provided to illustrate certain embodiments of the invention, but are not to be construed as limiting the invention and its equivalents as defined by the appended claims.

### EXAMPLE 1

Human whole blood samples were processed according to the invention and tested for amplification of a target nucleic acid sequence in conventional SDA and in thermophilic SDA. The blood samples were spiked with an *E. coli* strain carrying an integrated copy of the *M. tuberculosis* IS6110 (EC21) insertion element prior to sample processing. The IS6110 insertion element served as the post-processing target sequence for amplification.

Stock cultures of EC21 were prepared by culturing in 2 ml of LR broth at 37°C for 3 hours with shaking. The concentration of organisms was estimated by OD and the cultures were diluted in PBS or KPDG buffer to 10⁴, 10³, 500 or 250 organisms/ml. The PBS dilutions (100 µl) were plated in duplicate with a PBS-only control to confirm the organism count.

Whole blood or PBS (5 ml) was spiked with 100 µl of each EC21 dilution and added to 20 ml of lysis buffer (0.26% saponin in PBS) in a high speed centrifuge tube. TRITON X-100 (0.2-1%) was also evaluated as a lytic reagent. Positive controls (EC21 in KPDG) and negative controls (KPDG only) were also prepared. The tubes were mixed by inverting several times and centrifuged at 10,000 rpm for 20 min. at 4°C. The supernatants were carefully decanted leaving approximately 500 µl of residual fluid to avoid disturbing the EC21-containing pellet. The pellets were resuspended in the residual fluid and 1 ml PBS and transferred to 2 ml capped microcentrifuge tubes. The samples were centrifuged in the microcentrifuge for 3 min. at 13,000 xg at room temperature. Supernatants were removed with a micropipette and discarded, leaving approximately 100 µl of residual fluid with the pellet. The pellets were resuspended in 1 ml of KPDG and microcentrifuged as before, discarding the supernatants. The KPDG wash was repeated once.

The final pellet was resuspended in a total volume of 200 µl or 500 µl by addition of KPDG and Proteinase K (final concentration 0.5 mg/ml), estimating 100 µl residual volume of pellet and fluid. The resuspended sample was mixed by vortexing and incubated at 55°C in a hot air convection oven for 30 min. The temperature was then increased to 100°C for 15 min. to inactivate the protease. Timing for these incubations was begun when the air temperature reached the set temperature. Samples were then cooled, mixed by vortexing and stored at 4°C.

For thermophilic SDA, 0.5 ml EPPENDORF tubes were set up for the amplification reactions and the controls. The controls were *M. tuberculosis* genomic targets containing the IS6110 insertion element in 50 ng/µl human placental DNA. The IS6110 control targets were diluted in duplicate to 0, 10, 50 and 100 targets/5 µl of target diluent. The amplifications contained additional human DNA to bring the total amount of DNA in the reaction (human DNA + target) to 500 ng. Amplification primers (S) and bumper primers (B) were synthesized with the target binding sequences described by G. T. Walker, et al. *Nucl. Acids Res.* **20**, 1691-1696 (1992), substituting a BsoBI recognition site for the HincII recognition site. The components of the amplification reactions were as follows: 35 mM KPO₄, 0.1 mg/ml BSA, 0.2 mM dATP and dGTP, 0.5 mM dUTP, 1.4 mM α-thio-dCTP, 0.5 µM amplification primers, 0.05 µM bumper primers, 10% glycerol, 6 mM MgCl₂, 8 units BCA polymerase, 160 units BsoBI, 500 ng DNA (human placental DNA and target), and 0.716X NEB2 restriction enzyme buffer (New England Biolabs, added with enzymes).

Each tube contained either 25 µl of sample or 5 µl of the genomic control target. Prior to amplifcation, each sample was decontaminated as follows. After mixing by vortexing, samples were heated in a boiling water bath for 3 min. The samples were cooled to 55.5°C for several minutes in a heating block to allow equilibration to the set point. UDG was added (1 µ l of a 0.5 unit/µl stock solution), and the tubes were mixed and incubated for 5 min. in the heating block at 55.5°C. The UDG was then inactivated by addition of 2 units/reaction Ugi. The thermophilic SDA reaction was then allowed to proceed by mixing and incubating for 30 min. at 55.5°C with the cover on the heating block to reduce evaporation. Samples were mixed once during the amplification reaction, at 15 min. The amplification reaction was stopped by storage at 4°C until assay of the results.

SDA of the IS6110 target sequence and an internal control (signature) sequence at 40°C (conventional SDA) was performed in the adapter-mediated multiplex format essentially as described by G. T. Walker, et al. *Nucl. Acids Res.* **22**, 2670-2677 (1994). Amplification products generated in the thermophilic SDA and conventional SDA reactions were detected in a chemiluminescent assay as described by C. A. Spargo, et al. *Molec. Cell. Probes* **7**, 395-404 (1993), using 10 µl of each sample or control. Capture and detector probes for the IS6110 target sequence were as described. The capture probe for the signature sequence was the D_{control} probe described by Walker, et al. (1994), *supra* (Table I), with three biotin groups at the 5' end. The detector probe for the signature sequence was:
5'-TCAGACATCGTCGCT-AL2 (SEQ ID NO:1, AL2 = AminoLink)
Plates were incubated, uncovered, at 37°C for 45 min. for hybridization with the IS6110 and signature detector probes. Following the stringency washes and incubation with the LUMIPHOS (Lumigen, Inc., Detroi, MI) substrate, luminescence was read in a microtiter plate luminometer. Data was relayed to DELTASOFT software for statistics and the results were entered into a LOTUS spreadsheet for evaluation.

In the thermophilic SDA reactions, amplification in processed blood samples lysed with 0.26% saponin was equivalent to (i.e., 100% of) the positive controls. This corresponded to a sensitivity of 80 microorganisms/10 ml of whole blood or about two target sequences per amplification reaction. In conventional SDA, amplification after saponin lysis was somewhat less than 100% of the positive controls, but still resulted in a sensitivity of about 50 microorganisms/10 ml of processed blood. In general, lysis with 0.2-1% TRITON X-100 resulted in increasing inhibition of amplification as the concentration of TRITON increased. However, at 0.2% there was efficient lysis and a minimal reduction in amplification (about 50%) which was easily detected above background. As the TRITON concentration was increased up to 1%, inhibition of amplification increased but remained detectable above background. However, increasing TRITON X-100 in the lytic reagent also resulted in reduced viability of microorganisms in culture. Therefore, when TRITON is used to lyse blood which is also to be cultured, it is desirable to keep the concentration as low as possible, preferably at about 0.2%. Amplification was generally increased in samples resuspended in 500 µl as compared to samples resuspended in 200 µl. The effect of increased reaction volume was minimal in samples lysed with saponin and more pronounced in the samples lysed with TRITON, presumably due to the slightly inhibitory effect of TRITON.

## Claims

1. A method for processing whole blood for nucleic acid analysis of microorganisms consisting essentially of:
a) lysing an initial volume of whole blood by addition of saponin or 0.2-1.0% TRITON in saline;
b) centrifuging the lysed whole blood at 5,000-15,000 xg for 5-30 min. to form a cell debris pellet and a supernatant;
c) removing the supernatant from the cell debris pellet;
d) washing the cell debris pellet with a wash solution consisting essentially of saline, water, a physiological buffer or a buffer compatible with the nucleic acid analysis, and centrifuging at a speed sufficient to form a washed pellet which is resistant to loss of material during washing;
e) resuspending the washed pellet to a volume about 1/10-1/20 of the initial volume in a solution consisting essentially of saline, water, a physiological buffer or a buffer compatible with the nucleic acid analysis, and;
f) releasing nucleic acids from the microorganisms.

2. The method according to Claim 1 further comprising amplifying a target nucleic acid sequence of the microorganisms.

3. The method according to Claim 2 wherein the washed pellet is resuspended in KPDG buffer and the target nucleic acid sequence is amplified by Strand Displacement Amplification.

4. The method according to Claim 1 wherein the cell debris pellet is washed with about 0.5-1.5 ml of wash solution and centrifuged at about 8,000-15,000 xg for 1-10 min.

5. The method according to Claim 1 wherein the washing step is repeated.

6. The method according to Claim 1 wherein nucleic acids are released from the microorganisms by treatment with heat, protease, or mechanical disruption.

7. The method according to Claim 1 wherein the sample of whole blood is lysed by addition of saponin to about 0.2-0.5%.

8. A method for processing a sample of whole blood for nucleic acid analysis of microorganisms consisting essentially of:
a) lysing the sample of whole blood by addition of saponin to about 0.2-0.5%;
b) centrifuging the lysed whole blood sample at 5,000-15,000 xg for 5-30 min. to form a cell debris pellet and a supernatant;
c) removing the supernatant;
d) washing the cell debris pellet twice in saline, water, a physiological buffer or a buffer compatible with nucleic acid analysis, centrifuging between washes at 8,000-15,000 xg to form a washed pellet which is resistant to loss of material during washing;
d) resuspending the washed pellet in saline, water, a physiological buffer or a buffer compatible with the nucleic acid analysis;
e) treating the resuspended washed pellet with a protease, a nuclease, a chelating agent, or mechanical disruption, and;
f) heating the treated washed pellet of step (e) at 90-110°C for 1-20 min.

9. The method according to Claim 8 further comprising amplifying a target nucleic acid sequence released from the microorganism.

10. The method according to Claim 9 wherein the washed pellet is resuspended in KPDG buffer and the target nucleic acid sequence is amplified by Strand Displacement Amplification.


SEQUENCE LISTING

(1) GENERAL INFORMATION:

(i) APPLICANT: BECTON, DICKINSON AND COMPANY
(A) NAME: BECTON, DICKINSON AND COMPANY
(B) STREET: 1 Becton Drive
(C) CITY: Franklin Lakes
(D) STATE: NJ
(E) COUNTRY: US
(F) ZIP: 07417

(ii) TITLE OF INVENTION: SAMPLE PROCESSING METHOD FOR WHOLE BLOOD

(iii) NUMBER OF SEQUENCES: 1

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(v ) CURRENT APPLICATION DATA:
(A) APPLICATION NUMBER:
(B) FILING DATE:

(2) INFORMATION FOR SEQ ID NO:1:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:1:

TCAGACATCG TCGCT 15